(19) Europäisches Patentamt / European Patent Office / Office européen des brevets

(11) **EP 3 505 071 B1**

## (12) EUROPEAN PATENT SPECIFICATION

(45) Date of publication and mention of the grant of the patent:
**16.03.2022 Bulletin 2022/11**

(21) Application number: **17210882.1**

(22) Date of filing: **28.12.2017**

(51) International Patent Classification (IPC):
**A61B 8/06** (2006.01)    **A61B 8/00** (2006.01)
**A61B 8/08** (2006.01)    **G01S 15/89** (2006.01)

(52) Cooperative Patent Classification (CPC):
**A61B 8/06; A61B 8/4209; A61B 8/4281;**
**A61B 8/4477; A61B 8/488; G01S 15/8913;**
**G01S 15/8929; G01S 15/8984;** A61B 8/04;
A61B 8/15

(54) **ULTRASOUND PROBE FOR CONTINUOUS WAVE DOPPLER DEVICE AND USE OF THEREOF**

ULTRASCHALLSONDE FÜR CONTINUOUS-WAVE-DOPPLER-GERÄT UND DEREN VERWENDUNG

SONDE A ULTRASONS POUR DOPPLER DISPOSITIF POUR ONDES CONTINUES (CW) ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.07.2019 Bulletin 2019/27**

(73) Proprietor: **Przedsiebiorstwo Wdrozeniowo-Produkcyjne Sonomed sp. z o. o.**
**02-118 Warszawa (PL)**

(72) Inventors:
• **KARLOWICZ, Pawel**
**05-816 MICHALOWICE (PL)**
• **KARLOWICZ, Wlodzimierz**
**01-633 WARSZAWA (PL)**

(74) Representative: **Patpol Kancelaria Patentowa Sp. z o.o.**
**Nowoursynowska 162J**
**02-776 Warszawa (PL)**

(56) References cited:
| | |
|---|---|
| CN-A- 107 296 628 | JP-U- H0 418 504 |
| US-A- 4 357 944 | US-A- 5 562 098 |
| US-A1- 2006 241 460 | US-A1- 2006 259 260 |
| US-A1- 2007 167 783 | US-B1- 6 261 233 |

• **ROZBICKI JAKUB ET AL: "Doppler-based blood pressure measurement system for patients supported by a continuous-flow rotary left ventricular assist device", 2017 IEEE INTERNATIONAL ULTRASONICS SYMPOSIUM (IUS), IEEE, 6 September 2017 (2017-09-06), pages 1-4, XP033245100, DOI: 10.1109/ULTSYM.2017.8091990 [retrieved on 2017-10-31]**
• **DUNMIRE B ET AL: "Cross-beam vector Doppler ultrasound for angle-independent velocity measurements", ULTRASOUND IN MEDICINE AND BIOL, NEW YORK, NY, US, vol. 26, no. 8, 1 October 2000 (2000-10-01), pages 1213-1235, XP004295674, ISSN: 0301-5629, DOI: 10.1016/S0301-5629(00)00287-8**

## Description

TECHNICAL FIELD

The present invention relates to ultrasound continuous wave Doppler probe for easy blood flow registration in peripheral arteries, particularly in radial artery in patients with heart prosthesis. More particularly, the invention discloses an ultrasound probe for Doppler device for blood flow assessment which comprises at least three transducers mounted on a common carrier made preferably of material acoustically compatible relative to human body. This probe is particularly suitable for use in private domestic application by patients with heart prothesis. The invention relates also to use of said probe to the Doppler based NIBP measurements especially in patients with heart prosthesis named as left ventricular assist devices (LVADs).

BACKGROUND

The heart prosthesis - left ventricular assist devices (LVADs) have become the standard of care in patients with end-stage heart failure. It may be used as bridge to transplantation and destination therapy but sometimes it is treated by the patients also as a way to continue acceptable way of their life. The LVAD devices primarily working in pulse system now are more widely using continuous flow design.

Non-invasive blood pressure (NIBP) measured with different methods is not an exact reflection of the patient's true blood pressure as the ones obtained in the invasive way. There are many variables that can affect the results however NIBP provides very important diagnostic information.

The physiological and clinical significance of the arterial blood pressure (BP) has motivated many efforts to develop a reliable non-invasive blood pressure measurement technique. Three well known techniques have been used in non-invasive measurement of blood pressure (NIBP), namely: auscultation, oscillometry, and tonometry. Both the auscultation and oscillometry techniques use a standard inflatable arm cuff that occludes the artery - mostly brachial artery. The auscultatory technique determines the subject's systolic and diastolic pressures by monitoring certain Korotkoff sounds that occur as the cuff is slowly deflated. The oscillometry technique, on the other hand, determines these pressures, as well as the subject's mean pressure, by measuring actual pressure changes that occur in the cuff as the cuff is deflated. Two other means are applied in detection flow changes below the inflated and deflated cuff - the near infrared detection - Plethysmography and ultrasound Doppler methods.

Ultrasound blood flow detectors - simple Doppler devices are widely used in vascular diagnosis including the ankle brachial index (ABI) determination. The ABI is important way to diagnose peripheral vascular disease.

The techniques developed for the measurement of systolic BP are based on closing the artery under the cuff with pressure which is above the systolic value. The re-opening of the artery when the cuff pressure decreases below this value can be detected by a distal flow or pulse detection, The Doppler sounds from well-located and properly acoustic coupled transducer are very sensitive and accurate in such flow identification. The Doppler based NIBP measurements become very important for the patients with LVAD and specially in group with continuous flow devices. In that last case the blood flow pulsation may be not possible to identify by NIBP traditional devices. As published by Gregg M. Lanier at all, [Validity and Reliability of a Novel Slow Cuff-Deflation System for Non-invasive Blood Pressure Monitoring in Patients With Continuous-Flow Left Ventricular Assist Device] the Doppler assessments have excellent success rates, but cannot be performed by patients at home, requiring highly trained personal in the hospital setting.

There is a need to provide simple in use and reliable Doppler based device which may be easily used by shortly trained patient in any environment, including patient's home. The development of such device consists of two components: software to analyze Doppler sounds and ultrasound module with transducer assuring very easy positioning over the vessel (mainly radial artery) to get good flow signal which may be applied as a base for NIBP measurement.

A continuous wave (CW) is the simplest technology for detection of flow with ultrasound. This operation is based on two separate elements in transducer which are used in a way that one transmits the acoustic energy, the other receives echoes back. Electronics in CW devices require practically no user controls and settings, so only a correct positioning of the probe over the vessel is substantial. Physical phenomena associated with ultrasound waves are the reason to seek compromises in order to optimize equipment.

In CW Doppler ultrasound transducer, the sensitive area is where the effective beams (ultrasound propagation fields) of the two elements overlap. The best signal to noise background will be observed when the sensitive area is a close equivalent to cross-section of the vessel in which the blood flow is registered. On the other, hand the easy vessel localization and continuous coverage by ultrasound beam during small movements (muscle activity, inflated cuff influence) require to apply possible bigger sensitive area. For the untrained with Doppler diagnosis user, a reasonable compromise requires noticeable bigger sensitivity area than just vessel coverage area.

Another important factors are referring to the angle of the ultrasound beam passing through the media borders (like probe's front to the patients tissue) and between ultrasound beam direction and flow direction.

The Doppler shifted frequencies are obtained by comparison of the transmitted signal with the received signal. The basic equation of Doppler shifted frequency from which it is possible to calculate flow velocity may be presented as follows:

$$fDoppler = 2f_0 \cdot v_c \cdot \cos\Theta / c$$

where $v_c$ is the flow velocity , $f_0$ is the original transmit frequency, c is the sound velocity in the media, $\Theta$ is an angle between flow direction and ultrasound beam direction . Following the cosine function in this equation the highest Doppler frequency for certain flow will be with angle $\Theta = 0$ while it will disappear at $\Theta = 90$ degrees. In practice due other physical limitations the recommended Doppler angle values in medical diagnostics of superficial vessels with hand held transducer shall be between 45 and 60 degrees.

In the applications requiring positioning and fixing the transducer on patient's skin the wedge between the skin and the probe surface filled with ultrasound gel shall be replaced by the solid material. Requirements for this wedge material specifications consists of the low acoustic attenuation and sound velocity possible close to the value in the water (human body like).

Two piezoelectric elements, from which one operates as transmitting one and the second operates as receiving one, are widely used in continuous wave (CW) Doppler probes. Utilization of greater number thereof on common surface in ultrasound scanner probes is met. It is also known solution to apply the positioning material for transmitting and receiving elements of Doppler probe at the angle allowing to register flow signal when it is arranged as flat on body surface. It is used e.g. in flat probe or transesophageal probe for veterinary medicine applications ["Weterynaria w praktyce" vol.6, nr 7-8/2009].

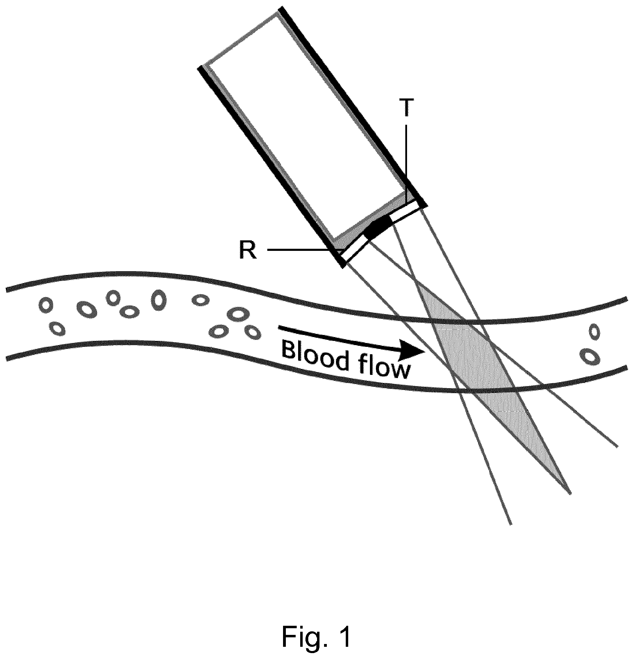

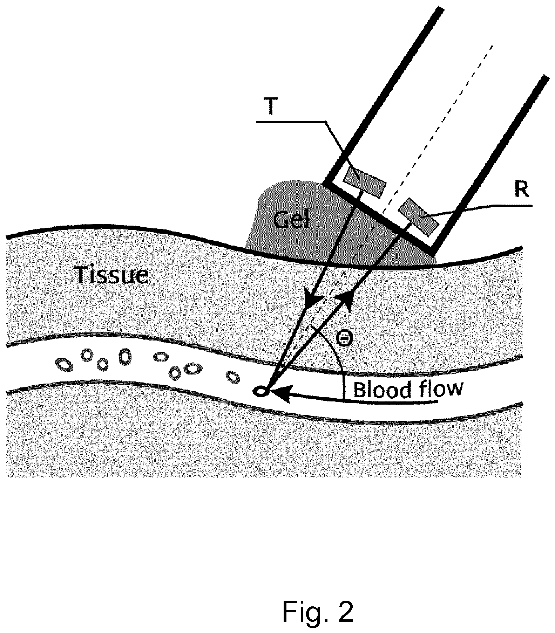

Figure 1 and Figure 2 show in schematic manner the ultrasound beam path in traditional CW probe with one transmitting transducer T and one receiving transducer R. Cross-hatched area, corresponding to the common part of ultrasound field path in Figure 1, represents the high sensitivity zone. It shall include the cross-section of blood vessel to obtain good Doppler signal.

The value of angle between direction of blood flow and direction of ultrasound wave as presented in Figure 2 is essential physical condition to obtain Doppler signal. Relation between Doppler frequency and flow velocity is associated among other things to the cosine function. At too large angle Doppler effect approaches zero. On the other hand, for the vessels, which path is typically approximately parallel relative to the skin surface, it is impossible to obtain small angle values because of ultrasound beam path between material before transducer and patient body (critical refraction angle). Thus, incidence angles of ultrasound beam on examined individual tissue border should be such that ultrasound beam incidence angles when the beam is refracted in tissue thereof are in the degrees range from 45° to 60° relative to the blood flow direction of the individual.

PRIOR ART

American patent document no. US 6346081 discloses an apparatus and method for determining the velocity of a fluid flowing through a lumen. The apparatus comprises a first diffraction grating transducer (DGT) responsive to a continuous wave (CW) input and operable in a first mode for producing a first signal beam at a first frequency and first phase, and in a second mode for producing a second signal beam at the first frequency and a second phase; a second diffraction grating transducer (DGT) operating as a receiver and coupled to the first diffraction grating transducer at a predetermined angle, the second diffraction grating transducer producing a first beam which intersects the first DGT first beam for receiving a first reflected signal associated with the first signal beam, and for producing a second beam which intersects the first DGT second beam for receiving a second reflected signal associated with the second signal beam.

US patent document no. US 8764663 discloses an apparatus and a method for transmitting a signal directed to a blood vessel via a Doppler ultrasound waveform apparatus, receiving, by the Doppler ultrasound waveform apparatus, a reflected signal from the blood vessel; and determining, by a processing device, whether the blood vessel is an artery or a vein based on a blood flow velocity in the blood vessel.

US patent document no. US 4582066 discloses an ultrasonic transducer having a transducer probe, for insonifying an organ or part thereof in a human patient from a position within the suprasternal notch thereof, wherein the transducer probe is in at least a partially obscured disposition within the notch vis-a-vis the operator thereof during insonification, is comprised of an elongate handle member with a proximal and a distal end, having a non-circular peripheral cross-sectional geometry including at least one longitudinal edge to yield a gripping surface providing the operator with the ability to manipulate the probe via tactile sensing of its position and a transducer probe extending from the distal end of, and generally normal to, the handle, wherein the probe has a generally arcuate cross-sectional geometry and a generally trapezoidal profile to enhance patient comfort during operator manipulation of the probe.

European patent document no. EP 458146 relates to a continuous wave driven ultrasound transducer for determining Doppler frequency shift in reflected ultrasound pressure waves wherein the transmitter and receiver sections are constructed of a composite core having a plurality of segments of piezoelectric material separated by acoustic suppression material.

American patent no. US 7549964 relates to a method of measuring blood flow through a blood vessel using a single quasi-continuous mode probe that can support multiple frequencies without increasing the probe tip size. A plurality of elements are provided in the probe tip. Each element emits ultrasound waves using a long pulsed signal with each element having a different resonant frequency. Each element also receives ultrasound energy in a continuous mode.

The further American patent no. US 6344024 relates to an ultrasound probe for use especially in medical diagnostics using ultrasonography. Said probe comprises an ultrasound sensor configured to generate an ultrasound send signal as a reaction to an electrical impulse and to receiving a receive signal reflected on an observation medium. Said ultrasound sensor is held in a supporting device and fitted with coupling and transmission devices for sending the transmission signal into the observation medium.

The document no. US 2007/167783 discloses an apparatus and method for generating selected audio signals to aid in the detection of certain conditions with the usage of ultrasound system. This document describes a probe comprising 6 receiving elements surrounding a central transmitter. The receivers are canted at an angle of 25 degrees, creating a "sensitive volume" where the transmit and receive beams overlap and within which moving blood will produce a Doppler signal in the receivers. The document no. US 2006/241460 relates to a blood rheology measurement device and method by use of an ultrasonic wave. Document no. JP H0418504 relates to utility model regarding an ultrasound diagnostic apparatus having enhanced safety in ultrasonic wave transmission to a living body. Document US 5562098 concerns a method of measuring blood flow velocity within a vessel, independent of probe angle, but using two Doppler signals from the same source. The publication of Rozbicki Jakub et all entitled "Doppler-based blood pressure measurement system for patients supported by a continuous-flow rotary left ventricular assist device" was published in 2017 by the Applicant's team and describes the system of single wave Doppler module to be used for a purpose of LVDAs patients. Said publication does not refer to any special design of any probes being used in cooperating with standard Doppler system. In contrary, it just evokes a necessity of elaborating a special probe designs what could be useful in LVADs patients.

All the Doppler probes known from the prior art which may be applied in NIBP measurements require trained medical personnel as an operator. It excludes practically the systematic everyday use of such measurement in a patient home during the rehabilitation. The probes according to the solutions known from the prior art when used by the not well trained person give not accurate ultrasound recording what excludes ability to evaluate NIBP.

In order to make the Doppler based NIBP measurements easy and fast the dedicated probe has been presently designed. The characteristics of this probe combines an enlarged sensitivity area and significant independence from the angle at which it is placed against the vessel on the patient's skin surface.

SUMMARY OF THE INVENTION

The object of the invention is a development the ultrasound probe for continuous wave Doppler device, fulfilling aforementioned requirements relative to obtaining simultaneously relatively large (enlarged) sensitivity area and relatively large range of positions on patient's body surface enabling Doppler signal registering.

The subject of invention is defined in independent claim 1. By providing a specific arrangement of walls, namely angles and acoustic beam path in wedge material the good independence of ability to detect Doppler flow signal from probe on the skin location is obtained. Moreover due to a pair of transducers working simultaneously the extended sensitivity area of Doppler detection is guaranteed.

Preferably according to the invention, the structure of said wedge member is symmetrical, and the transducers are mounted in axial symmetry system. By providing the symmetry in this construction, the path of the returning beam is optimized and the flexibility of probe positioning is better.

Preferably according to the invention, transducers arranged on a central and the side walls are formed as one or more transducers elements. Thanks to the possibility of implementing each transducer in one or in multiple pieces the availability on the market certain transducer shape is not limiting manufacturing of the probe.

Advantageously, a transducer arranged on the central wall of said wedge member passes through the plane defining symmetry axis of this wedge member. Preferably, transducers arranged on oblique walls are placed substantially symmetrically relative to the central wall of said wedge member whereas the oblique side walls are mutually convergent in axial symmetry.

According to the invention, a material of a wedge member constitutes a material having a defined sound velocity value, wherein for this determined value of sound velocity the inclination angles of said side walls and central wall, and also the convergence angle between said side walls, are calculated to obtain the angle between a direction of an ultrasound beam when it is refracted and a pre-determined blood flow direction in optimum range of 45 to 60 degrees.

The inclination angle A of said side walls, relative to the base of the wedge member is in the_range from 30 to 83, preferably from 37 to 82, more preferably from 44 to 81, and the most preferably from 47 to 49 degrees, and the inclination angle B of said central wall of this wedge member relative to its base is in the range from 5 to 35, preferably from 6 to 32, more preferably from 7 to 28, and the most preferably from 14 to 16 degrees, whereas the convergence angle C of the side walls in this wedge member is in the range from 3 to 26, preferably from 3,5 to 23, and more preferably from 4 to 21, and the most preferably from 9 to 11 degrees. A selection of angle values is dependent on sound velocity in a selected wedge material and resulting thereof beam deflection value on the border with patient's body.

In one variant of the probe according to the invention, the oblique side walls of the wedge can be symmetrical and suitable inclination angles A, B and C, depending on wedge material used, are in the ranges aforementioned above.

In one more variant of the probe according to the invention, transducers located on central wall and on the side walls of said wedge member, respectively, can be divided into two or more components.

In one variant of the probe according to the invention, transducers arranged on side walls of the wedge operate as transmitters, and transducer arranged on central wall operates as receiver.

In further variant of the probe according to the invention, transducers arranged on side walls operate as receivers, and transducer arranged on central wall operates as transmitter.

Another subject of the present invention relates to use of the above identified probe for the Doppler based NIBP measurements especially in patients with heart prosthesis named as left ventricular assist devices (LVADs).

BRIEF DESCRIPTION OF THE DRAWINGS

The invention is to be explained in more details below based on embodiments depicted in the following figures where:

Figure 1 is a schematic illustration of ultrasound beam path in prior art CW probe with one transmitting transducer T and one receiving transducer R;

Figure 2 is a schematic illustration according to the prior art of Θ angle between blood flow direction in patient body and ultrasound wave direction;

Figure 3 presents in a schematic perspective view of one preferred embodiment of an ultrasound probe according to the invention;

Figure 4 shows the view of the probe according to the invention presented generally in Fig. 3 in more detailed views, namely in side views - Figures 4a and 4b, respectively, and in the plane view of Fig. 4c and illustrates angles A, B, and C in wedge structure, respectively;

Figure 5 is a schematic presentation of extended sensitivity area for one of the embodiment of a probe according to the invention;

Figure 6 presents views 6a, 6b, 6c, 6d, 6e of another embodiment of the probe according to the invention;

Figure 7 shows actual distributions of ultrasound field intensity (registered by means of hydrophone in the field scanner) on selected planes transversal to the direction of ultrasound wave propagation for exemplary realization of the probe according to invention;

Figure 8 presents the flow velocity plots registered in the phantom by means of commercial flow meter equipped with standard flat probe and with exemplary probe according to the invention.

DETAILED DESCRIPTION OF THE PREFERRED EMBODIMENTS

Exemplary embodiments of the present invention will now be described in detail with reference to the accompanying drawings. The invention may however be embodied in many different forms and shall not be construed as being limited to the embodiments set forth herein. Rather these embodiments are provided so that this disclosure will be thorough and complete and will fully convey the scope of the invention to those skilled in the art. In the drawings, the shapes and dimensions may be exaggerated for clarity, and the same reference numerals will be used throughout to designate the same or like components.

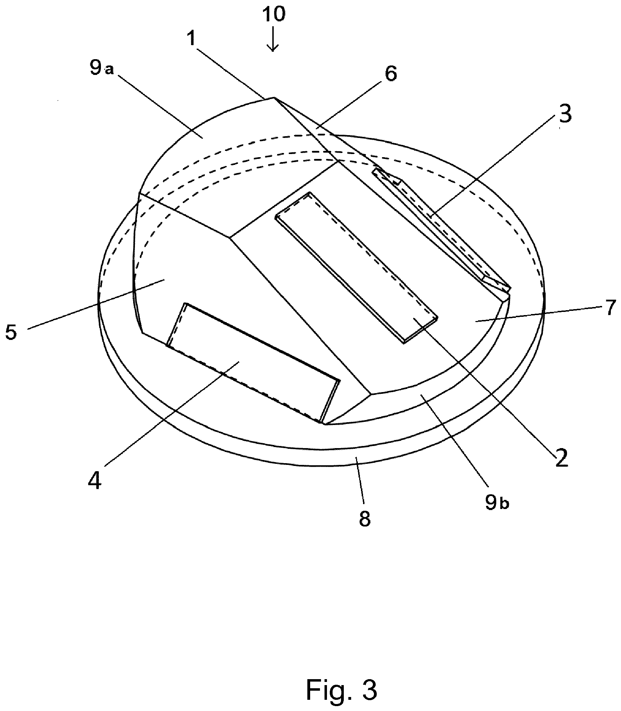

In Figures 3-4 and 5, the probe 10 according to the invention is presented. The probe 10 has a main body on which ultrasound transducers 2 and 3, 4 are arranged. This main body has a form of the common wedge member 1 encompassed by preferably symmetrical oblique side walls 5, 6 located at the angle A relative to the base plane 8 of the probe 10. In another embodiment these oblique side walls 5, 6 can be non-symmetrical. A wedge member 1 is encompassed from the top by upper wall 9a which passes forward into inclined central wall 7, and further, towards the base 8 into a curved face wall 9b. Central wall 7 is inclined relative to the wedge base at angle B. Transducers 3, 4 operating by example as the ultrasound beam transmitters are arranged on oblique side walls 5, 6 of said wedge member. On inclined front wall 7 the third transducer 2 is arranged then operating for example as the ultrasound beam signal receiver. The side walls 5, 6 of the wedge 1 on which transducers 3, 4 are arranged, except of an inclination relative to the probe face surface, are mutually convergent at angle C defined between a direction of side wall 5, 6 and the symmetry axis of the wedge. This results in significant difference of ultrasound beam direction for each pair - central transducer 2 with one of side transducer 3, 4.

Wedge member 1 is preferably mounted on substantially circular base 8. However, a base shape of said wedge 1 is not critical and other shapes thereof are possible, for example, a square shape, and also other variant of embodiment is likely possible in which the probe operates without having any base.

Transducers constitute electronic components, preferably piezoelectric ones, which can operate as ultrasound beam transmitters or as receivers of the signal, wherein at least two of them arranged on side walls of the wedge operate together. The piezoelectric transducers are typically in a shape of the plates which thickness corresponds the acoustic operating frequency while width and length are responsible for the shape of the ultrasound beam.

Wedge member 1 according to the invention can be made of plastic material preferably selected among acrylic glass, organic glass or plexiglass, polyetherketone of epoxy resins PEEK, polysulfone PSU, low density polyethylene, polystyrene "Lustrex" or crosslinked "Rexolite", preferably of polymethylpentene material known as TPX -, also known as poly(4-methyl-1-pentene).

The criteria for the choice of the wedge material consists simultaneously of the compatibility for the use in medical applications, low ultrasound acoustic waves attenuation and the expected range of the sound velocity in it. The possibility to permanently fasten, in practice to glue, the transducers elements namely piezoceramics with metal electrodes on their surfaces have to be considered.

By the way of example, in one variant of the probe 10 according to the invention where the oblique side walls 5, 6 of the wedge member 1 are symmetrical, and when said wedge member 1 is made of Lustrex polystyrene for which the sound velocity in material is 2032 m/s, the calculated angles in this wedge are in degrees: A=44°, B=14°, C=9°, respectively.

By the way of example, in one variant of the probe 10 according to the invention where the oblique side walls 5, 6 of the wedge member 1 are symmetrical, and when said wedge member 1 is made of plastic material poly(4-methyl-1-pentene) known under trade name TPX DX845 for which the sound velocity in material is 2150 m/s, the calculated angles for this wedge are in degrees: A=48°, B=15°, C=10°, respectively.

In another possible variant of the probe 10 according to the invention, the oblique side walls of the wedge can be non-symmetrical, and suitable inclination angles A, A', B and C, C' depending on wedge material used are in the general ranges afore mentioned above.

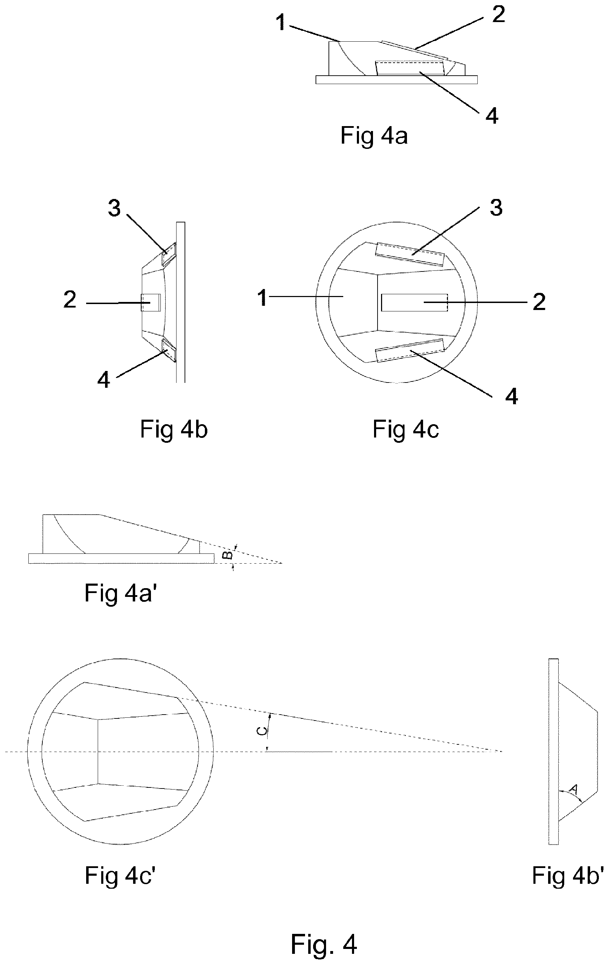

Figure 4 presents in more details an exemplary wedge member 1 with three transducers 2, 3, 4 for probe 10 according to the invention, in three different views: in the side views in Fig. 4a and Fig. 4b, and in the plan view in Fig. 4c, respectively, where side walls 5, 6 of the wedge are symmetrical, and upper wall 9a is parallel relative to the base 8 of the wedge-like member 1. Figure 4a depicts horizontally the side view of this wedge indicating (in figure 4a' respectively) angle B which determines the central wall 7 inclination angle relative to the base 8 of the wedge 1, whereas Figure 4b shows vertically another side view of said wedge, indicating angle A respectively in Figure 4b', corresponding to its top view from Figure 4c. Angle A indicates inclination angle of side wall 5, 6 of the wedge 1 relative to the base surface 8. Angle C is defined between side wall direction projected on the base and the wedge symmetry axis, what is showed in more details in Figure 4c'.

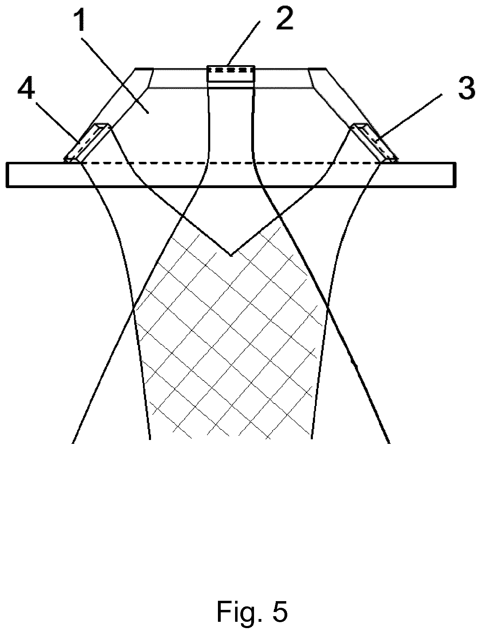

The inventive idea is presented in the best way in Figure 5 showing the high sensitivity area, in similar way to Figure 1, for the probe 10 in exemplary embodiment according to the invention. In the cross-section of the ultrasound beam path, the common range for the central transducer 2 and the sum of the side transducers 3, 4 corresponding to the high sensitivity area have been schematically cross-hatched. The arrangement whereby the two transducers have some overlap of their beams, resulting in a region of interest where the Doppler shifted signals may be detected (sensitivity area) was extended by the transducer shape and location on the wedge and mainly by the use of simultaneous operation of two transducer elements located in a distance and on sides of the third one.

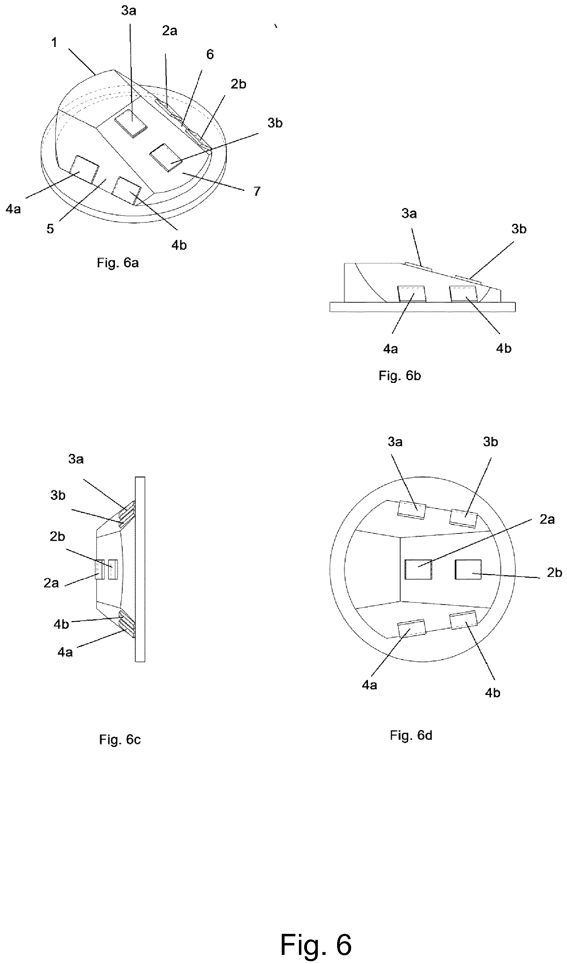

Figure 6 shows another one embodiment of the probe of the invention where transducers arranged on the oblique side walls 5, 6 and the central wall 7, are constituted of pairs of transducer elements such as 2a, 2b, 3a, 3b, 4a, 4b each having the smaller areas - what is showed schematically in the plan view in Figure 6d. Figure 6a shows two perspective views of said wedge, Figure 6b depicts horizontally side view of the same (indicating angle B in similar way to Fig. 4a'), whereas Figure 6c shows vertically another side view of said wedge (indicating angle A in similar way to Fig. 4b') corresponding to its top view from Figure 6d. On Figure 6d angle C can be indicated in similar way to Fig. 4c'.

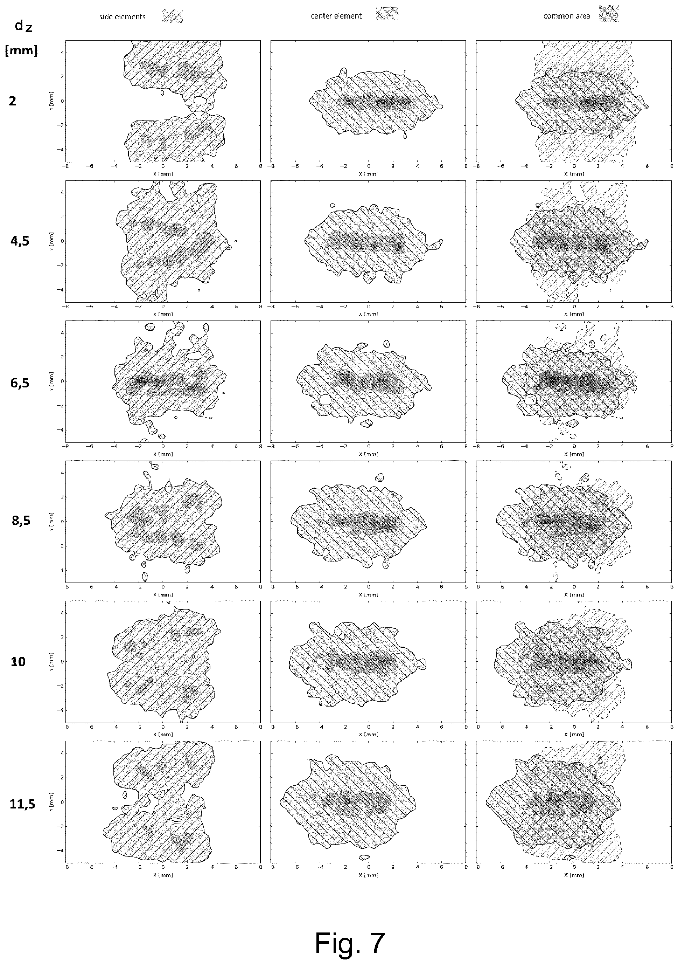

Figure 7 shows actual distributions of ultrasound field intensity (registered by means of hydrophone in the field scanner) on selected transversal planes in subsequent distances from the surface for exemplary realization of the probe 10 according to the invention. First column of distributions of ultrasound field intensity presents the field of the side transducers 3, 4 working together arranged on the side walls of said wedge 1 of the probe according to the invention. The second column of distributions of ultrasound field intensity presents the field of central transducers. In the third column of distributions of ultrasound field intensity the combination of the fields from the first and second column was shown, on which the double cross-hatching allows to distinguish the range of high sensitivity area. In subsequent lines in distributions of ultrasound field intensity, there is shown by means of intensity of cross-hatching the quantified intensity of the ultrasound beam field for subsequent planes distant from the surface by $d_z$ . The actual measurement data for exemplary embodiments confirm the substantial enlarging of the sensitivity area which, for interesting area of applications (potential path of artery under skin surface), is several times larger than that in the standard probe.

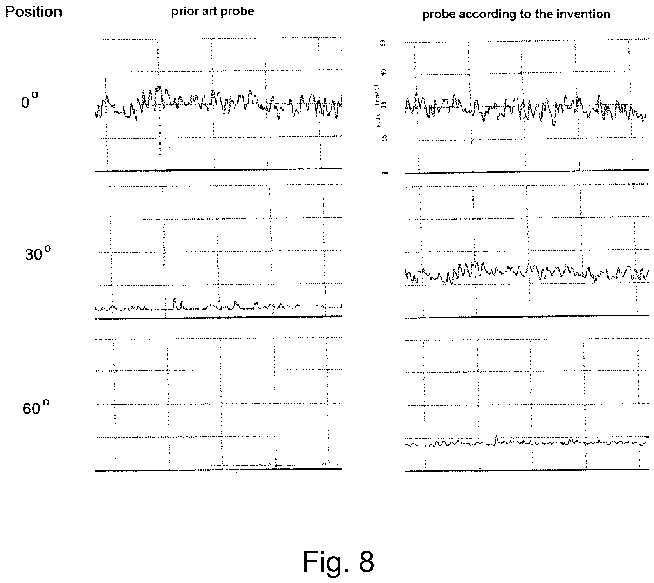

Figure 8 presents the recorded velocity of experimental measurements in the flow phantom. Using flow phantom in the form of silicon tube of 1, 5 mm diameter, inside which constant flow of the liquid (similar to blood) scattering ultrasounds placed 5 mm under the water surface in phantom vessel was forced. Then, the Doppler signal of velocity obtained using standard probe and made according to the invention, was registered by means of commercial flow meter. Both probes were arranged subsequently coaxially with flow direction, with 30 degree deviation from the axis and with 60 degree deviation from the axis. The significantly lesser dependence of ability for Doppler signal registering from position relative to the flow direction for the probe according to the invention has been proved.

In realization of the invention, the disadvantageous physical phenomenon of ultrasound wave reflection on the media border is essential and requiring to be taken into consideration. It is proportional to the ratio of acoustic impedances of the two media border. This impedance is expressed by means of the product of the density and acoustic wave velocity for the material. Since plastic materials applicable for wedge construction have acoustic wave velocities higher than that in the tissue, such plastic materials are advantageous, which have lower densities. In this regards, plastic material named TPX i.e. poly(4-methyl-1-pentene) is the most preferable.

It is an advantage of the invention that using the more than two transducing elements 2, 3, 4 constituting preferably piezoelectric transducers, which can be arranged such that they are inclined in two different planes (directions) relative to the base surface 8 of the wedge 1, allows to obtain the enlarged sensitivity area, and simultaneously the wider range of positions of the probe 10 as well, enabling a connection of the blood flow direction in the vessel with the ultrasound wave path direction in the manner ensuring to register Doppler signal of the flow thereof with maximum effectiveness.

A probe handling easiness during localizing of the blood flow signal due to enlarged sensitivity area is another advantage of the probe according to the invention, what is particularly useful in the case of less experienced operator as a patient with heart prosthesis. Essential advantage of the probe, due to reduced dependency on ultrasound beam angle, is also registering possibility of useful Doppler signal of blood flow in conditions of displacing as a result of small limb movements or displacement of anatomic structures beneath the probe during inflating the pressure gauge cuff .

EXAMPLES

Example 1

In one of examples of embodiment of the probe 10 according to the invention as material of wedge 1 the TPX -poly(4-methyl-1-pentene) plastic material has been used. The inclination angle A of the oblique side walls 5, 6 was 48°, and on the said side walls, symmetrically, piezoelectric transducers 3, 4, one for each wall, were arranged, which operate as ultrasound wave beam transmitters. The inclination of the central wall 7 of the wedge member 1 i.e. angle B, in this example of embodiment was 15°, and on this wall the piezoelectric transducer 2 (third one) operating as a receiver has been arranged. The convergence angle C of the side walls 5, 6 was 10°.

For the probe constructed in this manner the measurements of emitted ultrasound beams have been conducted (presented in Figure 7). In the 3D scanning system the acoustic pressure emitted by the probe was registered by means of hydrophone visualizing probe radiation field distribution on selected planes, and the essential enlargement of the sensitivity area has been confirmed.

Using the flow blood phantom in the form of silicon tube of 1, 5 mm diameter, inside which constant flow of the liquid (similar to blood) scattering ultrasounds had been forced, quality of Doppler signal obtained using standard probe and probe made according to the invention, was compared. The significantly lesser dependence of the latter on the position relative to the flow direction (Figure 8) has been proved.

After testing such constructed probe 10 in laboratory conditions on the bodies of volunteers, the high effectiveness and handling easiness at blood flow localizing in radial artery thereof has been proved.

While the present invention has been shown and described in connection with the exemplary embodiments, the scope of the invention is defined by the appended claims.

## Claims

1. Ultrasound probe (10) for continuous wave (CW) Doppler device for blood flow assessment in patient's peripheral arteries, particularly in radial artery in persons with heart prosthesis, which probe comprises three transducers mounted on a common carrier made preferably of material acoustically compatible relative to human body, wherein said common carrier is in the form of a wedge member (1) having a structure determined by two mutually oblique side walls (5, 6) and one central wall (7) inclined relative to a base surface of said wedge member, on which walls three transducers (2, 3, 4) are arranged, wherein one of said transducers (2) operates as receiver or transmitter and is arranged on said central wall (7), and the two remaining transducers (3, 4) operate together respectively as transmitters or receivers and are arranged one on each of the two oblique side walls (5, 6) on both sides of said central wall (7) of said wedge member (1), wherein said oblique side walls (5, 6) are mutually convergent under a convergence angle (C) having the range from 3 to 26 degrees, and said side walls (5, 6) have an inclination angle (A) relative to the base surface (8) of said wedge member (1) in the range from 30 to 83 degrees, and wherein an inclination angle (B) of the central wall (7) of said wedge member (1) relative to said base surface (8) is in the range from 5 to 35 degrees.

**2.** Probe according to according to claim 1, **characterized in that** the structure of said wedge member (1) is symmetrical.

**3.** Probe according to claim 1 or 2, **characterized in that** said transducer (2) on the central wall (7) and said transducers (3, 4) on the side walls (5, 6) are constituted of pairs of transducer elements (2a, 2b; 3a, 3b; 4a, 4b).

**4.** Probe according to any one of claims 1 - 3, **characterized in that** said transducers (2, 3) are arranged according to axial symmetry system.

**5.** Probe according to anyone of claims 1 - 4, **characterized in that** said transducer (2) arranged on the central wall passes through the plane determining symmetry axis of the wedge member (1).

**6.** Probe according to any one of claims 1 - 5, **characterized in that** said transducers (3, 4) are arranged on said oblique side walls (5, 6) substantially symmetrically relative to the central wall (7) of said wedge member (1).

**7.** Probe according to anyone of claims 1 - 6, **characterized in that** said oblique side walls (5, 6) are mutually convergent according to axial symmetry.

**8.** Probe according to any one of claims 1 - 7, **characterized in that** a material of the wedge member (1) constitutes the material having determined value of sound velocity, wherein for this determined value of sound velocity said inclination angles (A) of the two side walls (5, 6) and said inclination angle (B) of the central wall (2) and said convergence angle (C) of the two side walls (5, 6) are calculated to obtain an angle between an ultrasound beam direction after refraction and a pre-assumed direction of blood flow in optimum range of 45 to 60 degrees.

**9.** Use of the probe according to any one of claims 1-8, for the Doppler based NIBP measurements, especially in private domestic application for patients with heart prosthesis named as left ventricular assist devices (LVADs).

**Patentansprüche**

**1.** Ultraschallsonde (10) für eine Dauerstrich-Doppler-Vorrichtung zur Beurteilung des Blutflusses in den peripheren Arterien eines Patienten, insbesondere in der Radialarterie bei Personen mit einer Herzprothese, wobei die Sonde drei Wandler umfasst, die auf einem gemeinsamen Träger montiert sind, der vorzugsweise aus einem mit dem menschlichen Körper akustisch kompatiblen Material besteht, wobei der gemeinsame Träger die Form eines Keilelements (1) hat, das eine Struktur aufweist, die durch zwei gegenseitig schräge Seitenwände (5, 6) und eine Mittelwand (7), die geneigt relativ zu einer Basisfläche des Keilelements ist, bestimmt ist, wobei an diesen Wänden drei Wandler (2, 3, 4) angeordnet sind, wobei einer der Wandler (2) als Empfänger oder Sender arbeitet und an der Mittelwand (7) angeordnet ist, und zwei verbleibende Wandler (3, 4) zusammen als Sender bzw. Empfänger arbeiten und jeweils an jeder der zwei schrägen Seitenwänden (5, 6) auf beiden Seiten der Mittelwand (7) des Keilelements (1) angeordnet sind, wobei die schrägen Seitenwände (5, 6) unter einem Konvergenzwinkel (C) im Bereich von 3 bis 26 Grad zueinander konvergieren, und die Seitenwände (5, 6) einen Neigungswinkel (A) relativ zu einer Basisfläche (8) des Keilelements (1) im Bereich von 30 bis 83 Grad haben, und wobei ein Neigungswinkel (B) der Mittelwand (7) des Keilelements (1) relativ zu der Basisfläche (8) im Bereich von 5 bis 35 Grad liegt.

**2.** Sonde nach Anspruch 1, **dadurch gekennzeichnet, dass** die Struktur des Keilelements (1) symmetrisch ist.

**3.** Sonde nach Anspruch 1 oder 2, **dadurch gekennzeichnet, dass** der Wandler (2) an der Mittelwand (7) und die Wandler (3, 4) an den Seitenwänden (5, 6) aus Paaren des Wandlerelements (2a, 2b; 3a, 3b; 4a, 4b) gebildet sind.

**4.** Sonde nach einem der Ansprüche 1 bis 3, **dadurch gekennzeichnet, dass** die Wandler (2, 3) nach einem axialen Symmetriesystem angeordnet sind.

**5.** Sonde nach einem der Ansprüche 1 bis 4, **dadurch gekennzeichnet, dass** der an der Mittelwand angeordnete Wandler (2) durch die Ebene geht, die die Symmetrieachse des Keilelements (1) bestimmt.

**6.** Sonde nach einem der Ansprüche 1 bis 5, **dadurch gekennzeichnet, dass** die Wandler (3, 4) an den schrägen Seitenwänden (5, 6) im wesentlichen symmetrisch zur Mittelwand (7) des Keilelements (1) angeordnet sind.

**7.** Sonde nach einem der Ansprüche 1 bis 6, **dadurch gekennzeichnet, dass** die schrägen Seitenwände (5, 6) in axialer Symmetrie zueinander konvergent sind.

**8.** Sonde nach einem der Ansprüche 1 bis 7, **dadurch gekennzeichnet, dass** ein Material des Keilelements (1) das Material mit einem bestimmten Wert der Schallgeschwindigkeit darstellt, wobei für diesen bestimmten Wert der Schallgeschwindigkeit die Neigungswinkel (A) von zwei Seitenwänden (5, 6) und der Neigungswinkel (B) der Mittelwand (2) und der Konvergenzwinkel (C) von zwei Seitenwänden (5, 6) berechnet werden, um einen Winkel zwischen einer Ultraschallstrahlrichtung nach der Brechung und einer vorher angenommenen Richtung des Blutflusses im optimalen Bereich von 45 bis 60 Grad zu erhalten.

9. Verwendung der Sonde nach einem der Ansprüche 1 bis 8 für nicht-invasive Blutdruckmessungen auf Doppler-Basis, insbesondere in der häuslichen Anwendung bei Patienten mit einer Herzprothese, die als linksventrikuläre Hilfsgeräte (LVADs) bezeichnet werden.

**Revendications**

1. Sonde à ultrasons (10) pour dispositif Doppler à ondes continues (CW) pour l'évaluation du flux sanguin dans les artères périphériques du patient, en particulier dans l'artère radiale chez les personnes portant une prothèse cardiaque, laquelle sonde comprend trois transducteurs montés sur un support commun constitué de préférence d'un matériau acoustiquement compatible par rapport à corps humain, ledit support commun se présente sous la forme d'un membre en coin (1) ayant une structure déterminée par deux parois latérales mutuellement obliques (5, 6) et une paroi centrale (7), inclinée par rapport à une surface de base dudit membre en coin, sur lesquelles parois trois transducteurs (2, 3, 4) sont disposés, dans laquelle l'un desdits transducteurs (2) fonctionne comme récepteur ou émetteur et est disposé sur ladite paroi centrale (7), et des deux transducteurs restants (3, 4) fonctionnent ensemble respectivement comme émetteur ou récepteur et sont disposés l'une sur chaque des deux parois latérales obliques (5 , 6) des deux côtés de ladite paroi centrale (7) dudit membre en coin (1), dans laquelle lesdites parois latérales obliques (5, 6) sont mutuellement convergentes sous un angle de convergence (C) ayant la plage de 3 à 26 degrés, et lesdites parois latérales (5, 6) ayant un angle d'inclinaison (A) par rapport à la surface de base ( 8) dudit membre en coin (1) dans la plage de 30 à 83 degrés, et dans laquelle un angle d'inclinaison (B) de la paroi centrale (7) dudit membre en coin (1) par rapport à ladite surface de base (8) est dans la plage de 5 à 35 degrés.

2. Sonde selon la revendication 1, **caractérisée en ce que** la structure dudit membre en coin (1) est symétrique.

3. Sonde selon la revendication 1 ou 2, **caractérisée**

**en ce que** ledit transducteur (2) sur la paroi centrale (7) et lesdits transducteurs (3, 4) sur les parois latérales (5, 6) sont constitués de paire d'éléments de transduction (2a , 2b ; 3a, 3b ; 4a, 4b).

4. Sonde selon l'une quelconque des revendications 1 à 3, **caractérisée en ce que** lesdits transducteurs (2, 3) sont disposés selon un système de symétrie axiale.

5. Sonde selon l'une quelconque des revendications 1 à 4, **caractérisée en ce que** ledit transducteur (2) disposé sur le paroi centrale traverse le plan déterminant l'axe de symétrie du membre en coin (1).

6. Sonde selon l'une quelconque des revendications 1 à 5, **caractérisée en ce que** lesdits transducteurs (3, 4) sont disposés sur lesdites parois latérales obliques (5, 6) sensiblement symétriquement par rapport à la paroi centrale (7) dudit membre en coin (1).

7. Sonde selon l'une quelconque des revendications 1 à 6, **caractérisée en ce que** lesdites parois latérales obliques (5, 6) sont mutuellement convergentes selon la symétrie axiale.

8. Sonde selon l'une quelconque des revendications 1 à 7, **caractérisée en ce qu'**un matériau du membre en coin (1) constitue le matériau ayant une valeur déterminée de vitesse du son, dans laquelle pour cette valeur déterminée de vitesse du son lesdits angles d'inclinaison (A) des deux parois latérales (5, 6) et ledit angle d'inclinaison (B) de la paroi centrale (2) et ledit angle de convergence (C) des deux parois latérales (5, 6) sont calculés pour obtenir un angle entre une direction de faisceau d'ultrasons après réfraction et une direction présupposée du flux sanguin dans une plage optimale de 45 à 60 degrés.

9. Utilisation de la sonde selon l'une quelconque des revendications 1 à 8, pour les mesures NIBP basées sur Doppler, en particulier dans une application domestique privée pour les patients portant une prothèse cardiaque, appelée dispositifs d'assistance ventriculaire gauche (DAVG).

Fig. 1

Fig. 2

Fig. 3

Fig 4a

Fig 4b

Fig 4c

Fig 4a'

Fig 4c'

Fig 4b'

Fig. 4

Fig. 5

Fig. 6a

Fig. 6b

Fig. 6c

Fig. 6d

Fig. 6

Fig. 7

Position          prior art probe                 probe according to the invention

Fig. 8

**REFERENCES CITED IN THE DESCRIPTION**

**Patent documents cited in the description**

- US 6346081 B
- US 8764663 B
- US 4582066 A
- EP 458146 A
- US 7549964 B

- US 6344024 B
- US 2007167783 A
- US 2006241460 A
- JP H0418504 B
- US 5562098 A

**Non-patent literature cited in the description**

- *Weterynaria w praktyce,* 2009, vol. 6 (7-8